# FASCICULE DE BREVET EUROPEEN

(11) **EP 0 555 135 B1**
(45) Date de publication et mention de la délivrance du brevet: **14.08.1996**
(21) Numéro de dépôt: 93400247.8
(22) Date de dépôt: 02.02.1993
(51) Int. Cl.: C07K 1/18, C07K 14/75

(54) **Procédé de fabrication de fibrinogène de très haute pureté**
Verfahren zur Herstellung von hochreinem Fibrinogen
Process for preparing highly purified fibrinogen

(30) Priorité: 04.02.1992 FR 9201238
(43) Date de publication de la demande: 11.08.1993
(73) Titulaire: ASSOCIATION D'AQUITAINE POUR LE DEVELOPPEMENT DE LA TRANSFUSION SANGUINE ET DES RECHERCHES HEMATOLOGIQUES, F-33035 Bordeaux Cédex (FR)
(72) Inventeur: Dazey, Bernard, F-33000 Bordeaux (FR); Hamsany, Mohamed, F-33000 Bordeaux (FR); Enfedaque-Morer, Sylvia, F-33000 Bordeaux (FR); Vezon, Gérard, F-33670 Cursan (FR)
(74) Mandataire: Portal, Gérard

(56) Documents cités:
- EP-A- 0 383 645
- EP-A- 0 468 181
- WO-A-89/12065

## Description

La présente invention concerne essentiellement un procédé de fabrication de fibrinogène de très haute pureté, du fibrinogène de très haute pureté obtenu, ainsi qu'une composition pharmaceutique le contenant.

On sait que les indications du fibrinogène entrent dans la prévention d'hémorragie chez des malades souffrant de déficits quantitatifs ou qualitatifs en fibrinogène, d'insuffisance hépatocellulaire, de fibrinolyse et de coagulation intravasculaire disséminée (CIVD).

On connaît déjà dans l'état antérieur de la technique divers procédés de fabrication du fibrinogène par précipitation à la glycine comme décrit dans EP-A-0 383 234 ; ou par passage sur colonne d'héparine Sépharose comme décrit dans EP-A-0 359 593 = WO-A-89 1265 ; ou encore selon la méthode de COHN et a . (J. Am. Chem. Soc (1946), 68, page 459).

Ces concentrés de fibrinogène répondent aux normes de la pharmacopée européenne en présentant un taux de pureté d'au moins 60 %, ce qui conduit à un inconvénient lors de la reconstitution en solution du produit lyophilisé en raison d'un temps de reconstitution relativement long, pouvant atteindre 20 min.

Par ailleurs, le déposant a décrit dans le document FR-A-2 644 064 = EP-A-0 383 645, un procédé de fabrication de facteur antihémophilique (facteur VIIIc) ayant une très haute pureté permettant d'obtenir comme constituant secondaire non retenu du fibrinogène pour lequel aucune indication n'est décrite.

Encore, le document EP-A-0 468 181 décrit un procédé de purification de facteur VIII dans lequel il est simplement indiqué que le précipité restant issu du cryoprécipité dissous à froid après addition d'hydroxyde d'aluminium et de polyéthylèneglycol, peut servir de substance de départ pour la purification d'autres protéines incluant le fibrinogène et la colle de fibrine (page 3, lignes 32-33). Ce document ne contient aucune description ou suggestion comment obtenir du fibrinogène de haute pureté. Par ailleurs, dans l'invention qui sera décrite ci-après, le précipité à l'hydroxyde d'aluminium n'est pas récupéré.

La présente invention a pour but principal de résoudre le nouveau problème technique consistant en la fourniture d'un procédé de fabrication de fibrinogène ayant une très haute pureté, et ayant une vitesse de reconstitution du produit à l'état lyophilisé rapide, de préférence inférieure à 10 min et encore de préférence voisine de 5 min.

La présente invention a encore pour but de résoudre ce problème par un procédé de fabrication simple, permettant une excellente reproductibilité ainsi que de traiter des quantités importantes à l'échelle industrielle tout en étant très peu coûteux. De préférence, cette solution doit également permettre une inactivation virale.

La présente invention permet de résoudre ces problèmes techniques simultanément, d'une manière simple, peu coûteuse, utilisable à l'échelle industrielle en permettant de traiter des grandes quantités sans limitation de volume, tout en étant peu coûteuse.

Ainsi, selon un premier aspect, la présente invention fournit un procédé de fabrication de fibrinogène ayant une très haute pureté, caractérisé en ce qu'il comprend, à partir de cryoprécipité de plasma sanguin d'origine animale ou humaine, mis en solution aqueuse, le traitement de cette solution pour l'amener à une force ionique égale à celle d'une solution tampon ayant la composition suivante :

| | |
|---|---|
| NaCl | 120 mM |
| Tris | 20 mM |
| pH | 8,8 |

on fait passer cette solution de force ionique réglée sur une colonne d'échange d'anion comprenant un gel d'échange d'anion, par exemple sous forme de billes, à base d'agarose réticulé comprenant des groupements amine quaternaire, notamment au bout d'un petit bras espaceur, par exemple de type alkylène en C₁-C₆ relié aux billes d'agarose, en particulier à environ 6 % d'agarose ; et l'on procédé à une élution avec une première solution d'élution de même force ionique : NaCl 120 mM, Tris 20 mM, pH 8,8 ; ces conditions permettent d'adsorber au moins sélectivement le fibrinogène sur ladite colonne, puis on réalise la désorption sélective du fibrinogène, par un traitement avec une solution tampon d'élution sélective du fibrinogène de force ionique plus élevée, ayant la composition suivante :

| | |
|---|---|
| NaCl | 200 mM |
| Tris | 20 mM |
| pH | 8,8 |

et on recueille l'éluat ainsi obtenu contenant le fibrinogène sous forme hautement purifiée.

On observe que cet éluat à forte concentration en fibrinogène contient au moins 90 % de fibrinogène.

Selon une variante de réalisation avantageuse du procédé selon l'invention, on réduit la force ionique de l'éluat purifié contenant le fibrinogène en procédant à une diafiltration de préférence précédée d'une concentration. Cette diafiltration peut être avantageusement réalisée contre une solution tampon de conservation permettant une injection intraveineuse. Cette solution peut de préférence présenter la composition suivante :

| | |
|---|---|
| NaCl | 100 mM |
| Tris | 20 mM |
| L-arginine | 17 mM |
| L-lysine-HCl | 20 mM |
| pH | 7 |

L'éluat ainsi diafiltré et concentré peut ensuite être lyophilisé en obtenant ainsi du fibrinogène sous forme lyophilisée de très haute pureté, au moins égale à 90 %, immédiatement utilisable comme principe actif d'une composition pharmaceutique avantageusement pour les indications précédemment indiquées comme la prévention d'hémorragie chez des malades souffrant de déficits quantitatifs ou qualitatifs en fibrinogène, d'insuffisance hépatocellulaire, de fibrinolyse et de coagulation intravasculaire disséminée (CIVD).

Selon un mode de réalisation particulièrement avantageux du procédé selon l'invention, on utilise comme source de fibrinogène brut un cryoprécipité extrait de plasma sanguin d'origine animale ou humaine, de préférence humaine, que l'on met en solution dans de l'eau additionnée d'héparine.

Selon une variante de réalisation, la solution brute ainsi obtenue est débarrassée du facteur VIIIc par utilisation du procédé décrit dans le document antérieur du déposant FR-A-2 644 064 qui est incorporé ici par référence.

Selon une autre variante de réalisation, avant de séparer le facteur VIIIc, on procède à une inactivation virale, pour cela on peut tout d'abord traiter la solution brute avec l'hydroxyde d'aluminium (4 % en solution) dans la proportion de 10 à 15 % en volume par rapport au volume total de la solution de départ à traiter. On procède ensuite à une centrifugation pour séparer l'hydroxyde d'aluminium qui a piégé les facteurs vitamine K dépendants. On peut ensuite inactiver la solution avec un mélange d'inactivateurs à base de solvant/détergent comme décrit dans le document EP-A-0 131 740 ou US-A-4 540 573.

Selon une autre variante de réalisation avantageuse, avant de passer à l'étape de séparation du facteur VIIIc, de préférence par adsorption sur un gel d'échange d'ions de type anionique sous forme de billes d'agarose réticulé comportant des groupements amine quaternaire, notamment au bout d'un petit bras espaceur, par exemple de type alkylène en C₁-C₆ relié aux billes d'agarose, comme disponible commercialement sous le nom de Q Sépharose Fast Flow ^{R} de chez Pharmacia comme décrit dans FR-A-2 644 064, on procède à une diafiltration de la solution brute viralement inactivée à l'aide d'une solution tampon identique à celle servant à fixer le facteur VIIIc qui est décrite particulièrement à la revendication 11 de FR-A-2 644 064.

Cette solution tampon d'élution présente la composition suivante :

| | |
|---|---|
| NaCl | 250 mM |
| Tris | 20 mM |
| CaCl₂,2H₂0 | 10 mM |
| pH | 6,6 |

Ainsi, l'éluat, contenant la fraction non adsorbée, qui est débarrassé du facteur VIIIc qui a été adsorbé sélectivement sur le gel, contient Le fibrinogène qui est ensuite traité par le procédé selon l'invention précédemment décrit.

Selon une variante de réalisation avantageuse, le gel utilisé pour l'adsorption sélective du facteur VIIIc ou pour la purification du fibrinogène, est le même, ce qui facilite le procédé industriel de purification du fibrinogène.

Selon une autre variante de réalisation, permettant la conservation de la solution de fibrinogène de très haute pureté, on procède à une filtration stérilisante sur filtre stérilisant ayant une dimension de pores de diamètre à 0,22 µm, puis La solution stérilisée est introduite dans des flacons adéquats, de préférence en vue d'une lyophilisation. Avantageusement, on introduit un volume de solution de fibrinogène de très haute pureté de manière à obtenir un produit Lyophilisé pesant environ 1,5 à 2 g par flacon. Ce lyophilisat peut ensuite être repris pour l'injection intraveineuse avec 100 ml d'eau PPI.

D'autres buts, caractéristiques et avantages de l'invention apparaîtront clairement à la lumière de la description explicative qui va suivre faite en référence à un exemple le réalisation actuellement préféré donné simplement à titre d'illustration et qui ne saurait donc en aucune façon limiter la portée de l'invention. Dans cet exemple, les pourcentages sont donnés en poids, sauf indication contraire.

### Exemple de l'invention

9 kg de cryoprécipité issu de 900 l de plasma sanguin humain sont mis en solution dans 3,2 fois son volume d'eau osmosée à 3 UI d'héparine par millilitre d'eau à température du laboratoire sous agitation pendant 2 h.

Après mesure du volume final, on additionne à cette solution de l'hydroxyde d'aluminium à 4 % en solution aqueuse, à raison de 15 % en volume par rapport au volume total de cette solution. On laisse sous agitation pendant 5 min, puis on centrifuge à 6 000 G pour séparer l'hydroxyde d'aluminium de la solution.

Ensuite, on procède à une diafiltration de la solution brute contenant le facteur VIIIc ainsi que le fibrinogène contre une solution tampon qui sera ensuite utilisée pour l'équilibration et l'élution du gel de Q Sépharose Fast Flow ^{R} utilisé pour adsorber sélectivement le facteur VIIIc ayant la composition suivante :

| | |
|---|---|
| NaCl | 250 mM |
| Tris | 20 mM |
| CaCl₂ | 10 mM |
| pH | 6,6 |

La membrane servant à la diafiltration à un seuil de coupure de 100 kD (kilo Daltons).

Il est réalisé ensuite une concentration à 30 l de la solution brute de facteur VIIIc et de fibrinogène. On vérifie que cette solution concentrée présente sensiblement la même force ionique que la solution tampon servant à adsorber le facteur VIIIc sur le gel. La quantité de gel est, par exemple, de 100 l. Après élution du gel avec la solution tampon, on a ainsi adsorbé sélectivement le facteur VIIIc alors que la fraction protéique non retenue contient entre autre le fibrinogène à une concentration de 1,5 à 2 g/l ainsi que les inactivateurs et présente un volume de 150 à 200 l.

On sépare sélectivement le fibrinogène par le procédé selon l'invention de la manière suivante : on procède tout d'abord à une concentration de cette solution à 30 l avec une membrane à seuil de coupure de 100 kD, puis une diafiltration contre une solution tampon caractérisée par :

| | |
|---|---|
| NaCl | 120 mM |
| Tris | 20 mM |
| pH | 8,8 |

30 l de gel Q Sépharose Fast Flow® (PHARMACIA) préalablement équilibré par cette même solution tampon vont servir à fixer entre autre le fibrinogène, mais ne vont pas retenir l'albumine et les inactivateurs. Quand la densité optique du filtrat est retombée à la ligne de base, on élue le gel avec le même tampon après avoir augmenté sa force ionique par addition de NaCl à une concentration finale de 200 mM. Ce tampon d'élution sélectif du fibrinogène a donc la composition suivante :

| | |
|---|---|
| NaCl | 200 mM |
| Tris | 20 mM |
| pH | 8,8 |

La fraction du fibrinogène recueilli avec un rendement de 60 % par rapport à la quantité de fibrinogène injecté, est ensuite concentrée et diafiltrée contre un tampon présentant la composition suivante :

| | |
|---|---|
| NaCl | 100 mM |
| Tris | 20 mM |
| L-arginine | 17 mM |
| L-lysine-HCl | 20 mM |
| pH | 7 |

La concentration en fibrinogène est alors de 30 g/l environ ; on procède enfin à une filtration stérilisante de la solution de fibrinogène sur une membrane stérilisante de 0,22 µm de diamètre. Le produit concentré est réparti en 65 ml dans des flacons, puis lyophilisé.

## Revendications

1. Procédé de fabrication de fibrinogène ayant une très haute pureté, caractérisé en ce qu'il comprend, à partir de cryoprécipité de plasma sanguin d'origine animale ou humaine, mis en solution aqueuse, le traitement de cette solution jour l'amener à une force ionique égale à celle d'une solution tampon ayant la composition suivante :
| | |
|---|---|
| NaCl | 120 mM |
| Tris | 20 mM |
| pH | 8,8 |
on fait passer cette solution de force ionique réglée sur une colonne d'échange d'anion comprenant un gel d'échange d'anion, par exemple sous forme de billes, à base d'agarose réticulé comprenant des groupements amine quaternaire, notamment au bout d'un petit bras espaceur, par exemple de type alkylène en C₁-C₆ relié aux billes d'agarose, en particulier à environ 6 % d'agarose ; et l'on procède à une élution avec une première solution d'élution de même force ionique : NaCl 120 mM, Tris 20 mM, pH 8,8 ; ces conditions permettent d'adsorber au moins sélectivement le fibrinogène sur ladite colonne, puis on réalise la désorption sélective du fibrinogène, par un traitement avec une solution tampon d'élution sélective du fibrinogène de force ionique plus élevée, ayant la composition suivante :
| | |
|---|---|
| NaCl | 200 mM |
| Tris | 20 mM |
| pH | 8,8 |
et on recueille l'éluat ainsi obtenu contenant le fibrinogène sous forme hautement purifiée.

2. Procédé selon la revendication 1, caractérisé en ce qu'on réduit la force ionique de l'éluat purifié contenant le fibrinogène en procédant à une diafiltration de préférence précédée d'une concentration ; cette diafiltration étant avantageusement réalisée contre une solution tampon de conservation permettant une injection intraveineuse.

3. Procédé selon la revendication 2, caractérisé en ce que la solution tampon de conservation présente la composition suivante :
| | |
|---|---|
| NaCl | 100 mM |
| Tris | 20 mM |
| L-arginine | 17 mM |
| L-lysine-HCl | 20 mM |
| pH | 7 |

4. Procédé selon l'une des revendications 1 à 3, caractérisé en ce que l'éluat diafiltré est concentré et lyophilisé en obtenant ainsi du fibrinogène sous forme lyophilisée de très haute pureté, au moins égale à 90 %, immédiatement utilisable comme principe actif d'une composition pharmaceutique.

5. Procédé selon l'une des revendications 1 à 4, caractérisé en ce qu'on utilise comme source de fibrinogène brut un cryoprécipité extrait de plasma sanguin d'origine animale ou humaine, de préférence humaine, que l'on met en solution dans de l'eau additionnée d'héparine.

6. Procédé selon la revendication 5, caractérisé en ce que la solution aqueuse de cryoprécipité a subi une étape d'inactivation virale, par exemple par l'emploi d'un mélange de TnBP/TWEEN en observant la proportion relative de 0,3 % en TnBP et 1 % en TWEEN par rapport au volume de la solution à inactiver.

7. Procédé selon la revendication 6, caractérisé en ce que la solution brute obtenue est débarrassée du facteur VIIIc par adsorption sur un gel d'échange d'anion de type anionique sous forme de billes d'agarose réticulé comportant des groupements amine quaternaire, notamment au bout d'un petit bras espaceur, par exemple de type alkylène en C₁-C₆ relié aux billes d'agarose.

8. Procédé selon la revendication 7, caractérisé en ce qu'on utilise comme solution tampon d'élution une solution présentant la composition suivante :
| | |
|---|---|
| NaCl | 250 mM |
| Tris | 20 mM |
| CaCl₂,2H₂0 | 10 mM |
| pH | 6,6 |

9. Procédé selon la revendication 7 ou 8, caractérisé en ce que l'éluat, contenant la fraction non adsorbée, qui est débarrassé du facteur VIIIc qui a été adsorbé sélectivement sur le gel, contient un fibrinogène qui est ensuite traité selon l'une quelconque des revendications précédentes.

10. Procédé selon L'une quelconque des revendications précédentes, caractérisé en ce que le gel utilisé pour l'adsorption sélective du facteur VIIIc ou pour la purification du fibrinogène est le même.

## Patentansprüche

1. Verfahren zur Herstellung von Fibrinogen mit sehr hoher Reinheit, dadurch gekennzeichnet, daß es, ausgehend von einem Kryopräzipitat von Blutplasma tierischen oder menschlichen Ursprungs in wässeriger Lösung und der Behandlung dieser Lösung, um ihr eine Ionenstärke zu verleihen, die gleich jener einer Pufferlösung mit der folgenden Zusammensetzung:
| | |
|---|---|
| NaCl | 120 mM |
| Tris | 20 mM |
| pH | 8,8 |
ist, das Aufbringen dieser Lösung mit geregelter Ionenstärke auf eine Anionenaustauschersäule, die ein Anionenaustauscher-Gel, beispielsweise in Form von Kügelchen, auf Basis vernetzter Agarose mit quaternären Amingruppen, insbesondere am Ende eines kurzen Zwischenstücks, beispielsweise vom C₁-C₆-Alkylen-Typ, verbunden mit den Agarosekügelchen, insbesondere etwa 6 % Agarose, enthält; das Durchführen einer Elution mit einer ersten Elutionslösung der gleichen Ionenstärke: NaCl 120 mM, Tris 20 mM, pH 8,8; wobei diese Bedingungen eine zumindest selektive Adsorption des Fibrinogens auf der Säule ermöglichen; das anschließende Durchführen der selektiven Desorption des Fibrinogens durch Behandlung mit einer für Fibrinogen selektiven Elutions-Pufferlösung mit höherer Ionenstärke, die die folgende Zusammensetzung aufweist:
| | |
|---|---|
| NaCl | 200 mM |
| Tris | 20 mM |
| pH | 8,8; |
und das Sammeln des so erhaltenen Eluats, das Fibrinogen in hoch gereinigter Form enthält, umfaßt.

2. Verfahren nach Anspruch 1, dadurch gekennzeichnet, daß die Ionenstärke des gereinigten, Fibrinogen enthaltenden Eluats durch eine Diafiltration, der vorzugsweise eine Konzentration vorausgeht, reduziert wird; wobei diese Diafiltration vorteilhaft gegen eine Konservierungs-Pufferlösung, die eine intravenöse Injektion ermöglicht, durchgeführt wird.

3. Verfahren nach Anspruch 2, dadurch gekennzeichnet, daß die Konservierungs-Pufferlösung die folgende Zusammensetzung aufweist:
| | |
|---|---|
| NaCl | 100 mM |
| Tris | 20 mM |
| L-Arginin | 17 mM |
| L-Lysin-HCl | 20 mM |
| pH | 7. |

4. Verfahren nach einem der Ansprüche 1 bis 3, dadurch gekennzeichnet, daß das diafiltrierte Eluat konzentriert und gefriergetrocknet wird, wobei so Fibrinogen in gefriergetrockneter Form mit sehr hoher Reinheit, zumindest gleich 90 %, erhalten wird, das unverzüglich als aktiver Bestandteil einer pharmazeutischen Zusammensetzung verwendbar ist.

5. Verfahren nach einem der Ansprüche 1 bis 4, dadurch gekennzeichnet, daß als Quelle von rohem Fibrinogen ein aus Blutplasma tierischen oder menschlichen Ursprungs, vorzugsweise menschlichen Ursprungs, extrahiertes Kryopräzipitat verwendet wird, das in Wasser, dem Heparin zugesetzt wurde, gelöst wird.

6. Verfahren nach Anspruch 5, dadurch gekennzeichnet, daß die wässerige Lösung des Kryopräzipitats einem Schritt viraler Inaktivierung, beispielsweise durch die Verwendung einer Mischung von TnBP/TWEEN, unterworfen wurde, wobei das relative Verhältnis von 0,3 % TnBP zu 1 % TWEEN in bezug auf das Volumen der zu inaktivierenden Lösung eingehalten wird.

7. Verfahren nach Anspruch 6, dadurch gekennzeichnet, daß der Faktor VIIIc von der erhaltenen rohen Lösung durch Adsorption auf einem Anionenaustauscher-Gel vom anionischen Typ in Form vernetzter Agarosekügelchen mit quaternären Amingruppen, insbesondere am Ende eines kurzen Zwischenstücks, beispielsweise vom C₁-C₆-Alkylen-Typ, verbunden mit den Agarosekügelchen, entfernt wird.

8. Verfahren nach Anspruch 7, dadurch gekennzeichnet, daß als Elutions-Pufferlösung eine Lösung mit der folgenden Zusammensetzung verwendet wird:
| | |
|---|---|
| NaCl | 250 mM |
| Tris | 20 mM |
| CaCl₂,2H₂O | 10 mM |
| pH | 6,6. |

9. Verfahren nach Anspruch 7 oder 8, dadurch gekennzeichnet, daß das Eluat, das die nicht-adsorbierte Fraktion enthält, die frei vom Faktor VIIIc ist, der selektiv am Gel adsorbiert wurde, ein Fibrinogen enthält, das anschließend nach einem der vorhergehenden Ansprüche behandelt wird.

10. Verfahren nach einem der vorhergehenden Ansprüche, dadurch gekennzeichnet, daß das zur selektiven Adsorption des Faktors VIIIc oder zur Reinigung des Fibrinogens verwendete Gel das gleiche ist.

## Claims

1. Method for producing fibrinogen having very high purity, characterized in that it comprises, starting with cryoprecipitate of blood plasma of human or animal origin brought into aqueous solution, treatment of this solution to bring it to an ionic strength equal to that of a buffer solution having the following composition:
| | |
|---|---|
| NaCl | 120 mM |
| Tris | 20 mM |
| pH | 8.8 |
this solution of adjusted ionic strength is passed through an anion exchange column comprising an anion exchange gel, for example in bead form, based on crosslinked agarose comprising quaternary amine groups, in particular at the end of a small spacer arm, for example of the C₁-C₆ alkylene type attached to the agarose beads, especially containing approximately 6% of agarose; and an elution is performed with a first elution solution of the same ionic strength: 120 mM NaCl, 20 mM Tris, pH 8.8; these conditions enable at least the fibrinogen to be selectively adsorbed on the said column, selective desorption of the fibrinogen is then carried out by treatment with a buffer solution for selective elution of the fibrinogen, of higher ionic strength, having the following composition:
| | |
|---|---|
| NaCl | 200 mM |
| Tris | 20 mM |
| pH | 8.8 |
and the eluate thereby obtained, containing fibrinogen in highly purified form, is collected.

2. Process according to Claim 1, characterized in that the ionic strength of the purified eluate containing fibrinogen is reduced by performing a diafiltration, preferably preceded by a concentration; this diafiltration being advantageously carried out against a storage buffer solution permitting intravenous injection.

3. Method according to Claim 2, characterized in that the storage buffer solution possesses the following composition:
| | |
|---|---|
| NaCl | 100 mM |
| Tris | 20 mM |
| L-Arginine | 17 mM |
| L-Lysine.HCl | 20 mM |
| pH | 7 |

4. Method according to one of Claims 1 to 3, characterized in that the diafiltered eluate is concentrated and lyophilized, fibrinogen thereby being obtained in lyophilized form of very high purity, equal to at least 90%, which can be used immediately as active principle of a pharmaceutical composition.

5. Method according to one of Claims 1 to 4, characterized in that a cryoprecipitate extracted from blood plasma of animal or human origin, preferably of human origin, is used as a source of crude fibrinogen, the cryoprecipitate being dissolved in water to which heparin is added.

6. Method according to Claim 5, characterized in that the aqueous solution of cryoprecipitate has undergone a step of viral inactivation, for example by the use of a TnBP/TWEEN mixture, adhering to the relative proportion of 0.3% of TnBP and 1% of TWEEN relative to the volume of the solution to be inactivated.

7. Method according to Claim 6, characterized in that the crude solution obtained is freed from factor VIIIc by adsorption on an anionic exchange gel of the anionic type in the form of beads of crosslinked agarose containing quaternary amine groups, in particular at the end of a small spacer arm, for example of the C₁-C₆ alkylene type attached to the agarose beads.

8. Method according to Claim 7, characterized in that a solution possessing the following composition:
| | |
|---|---|
| NaCl | 250 mM |
| Tris | 20 mM |
| CaCl₂.2H₂O | 10 mM |
| pH | 6.6 |
is used as elution buffer solution.

9. Method according to Claim 7 or 8, characterized in that the eluate containing the unadsorbed fraction, which eluate is freed from the factor VIIIc which has been adsorbed selectively on the gel, contains a fibrinogen which is then treated according to any one of the preceding claims.

10. Method according to one of the preceding claims, characterized in that the gel used for the selective adsorption of factor VIIIc or for the purification of fibrinogen is the same.
